# EUROPEAN PATENT SPECIFICATION

(11) **EP 0 845 970 B1**
(45) Date of publication and mention of the grant of the patent: **26.01.2000**
(21) Application number: 96927224.4
(22) Date of filing: 06.08.1996
(51) Int. Cl.: A61J 1/00

(54) **BAG FOR CONTAINING A STERILE MEDICAL SOLUTION AND METHOD OF MIXING A STERILE MEDICAL SOLUTION**
BEUTEL FÜR EINE STERILE MEDIZINISCHE LÖSUNG UND VERFAHREN ZUM MISCHEN SOLCHER LÖSUNG
SAC DESTINE A CONTENIR UNE SOLUTION MEDICALE STERILE ET PROCEDE DE MELANGE D'UNE SOLUTION MEDICALE STERILE

(30) Priority: 08.08.1995 SE 9502769
(43) Date of publication of application: 10.06.1998
(73) Proprietor: GAMBRO AB, 103 91 Stockholm (SE)
(72) Inventor: WIESLANDER, Anders, S-222 38 Lund (SE); CAPPELLI, Giorgio, I-16145 Genova (IT)
(74) Representative: Asketorp, Göran
(86) International application number: PCT/SE96/00989
(87) International publication number: WO 97/05852

(56) References cited:
- EP-A- 0 442 406
- US-A- 3 187 750
- US-A- 4 403 992

## Description

### AREA OF THE INVENTION

The present invention relates to a bag for containing a sterile medical solution, comprising glucose or glucose-like compounds, for example a solution for peritoneal dialysis, or a nutritional solution.

### PRIOR ART

The present invention starts from what is disclosed in WO 93/09820, relating to a system where the glucose or glucose-like compounds are included in a first package at a high concentration whilst the remaining compounds are separately packed in a second package. The packages are preferably combined into a single, two compartment bag. The packages, or bag, are heat sterilized, preferably autoclaved in a conventional way. The high glucose concentration in the first package protects the glucose or glucose-like compounds from deterioration, which otherwise results in the formation of toxic compounds. Other factors influencing upon the formation of toxic compounds from the glucose or glucose-like compounds are low pH, absence of electrolytes, absence of oxygen contents, short exposure times (at higher temperature) etc.

Bags comprising several compartments are shown in, for example, US Patent 4 403 992 disclosing a bag device for continuous peritoneal dialysis having two chambers one being a dialysis liquid chamber and one being a disinfectant chamber. First the disinfectant is passed through the respective ducts, whereupon the dialysis liquid is passed through the same duct.

Another bag having several chambers is disclosed DE-C1-41 07 223. This bag has a specific frangible web between the chambers being openable for mixing the contents of the two chambers.

Another double compartment container is disclosed in EP-A1-619 998. This container includes a peelable seal constructed from a multilayer film.

A multicompartment flexible bag is disclosed in DE-A1-38 30 630 comprising a clamp dividing the bag into two compartments. By removing the clamp, the contents of the compartments can be mixed.

US-A-3 520 471 discloses a sealed flexible container for carrying fluids particularly collecting, storing and dispensing blood. The container has an inclined end seal for improving the possibility to empty the container.

US-A-4 496 046 discloses a multiple chamber container for the medical field such as nutrition solutions. The bag is composed of two or three compartments for mixing different solutions.

### DISCLOSURE OF THE INVENTION

The object of the present invention is to improve the bag shown in WO 93/09820 to make it more convenient to use, to make the mixing of the two liquids more efficient and to adapt the bag to easy and convenient construction and use by the end consumer and improve the logistic handling and storing of the bag.

These objects are met by a bag as defined in the appended patent claims.

### SHORT DESCRIPTION OF THE DRAWINGS

Further objects, features and advantages of the present invention will appear from the following detailed description of preferred embodiments of the invention shown on the enclosed drawings.

Fig. 1 is a plan view of a bag according to WO 93/09820 showing the prior art.

Fig. 2 is a plan view of a first embodiment of the bag according to the present invention.

Fig. 3-7 are plan views of further embodiments of the bag according to the invention.

### DESCRIPTION OF PREFERRED EMBODIMENTS

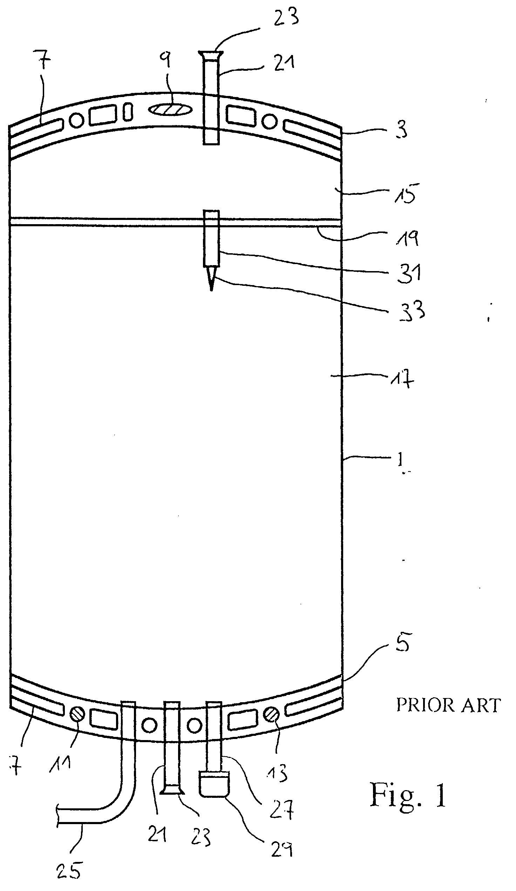

Fig. 1 is a plan view of a flexible bag according to WO 93/09820. The bag is intended to include a solution for peritoneal dialysis and has a size of approximately two and a half litres.

The bag is made from a continuous tube of a plastics material, which is sealed at both ends by sealing borders 3,5.

As shown in Fig. 1 each sealing border comprises several embossments 7 and apertures 9,11,13. The embossments 7 enhance the stability of the border 3. The aperture 9 in the upper border 3 is intended for hanging the bag during use and the apertures 11,13 in the lower border 5 are for fixation of the bag during the manufacturing operation.

The bag is divided into two compartments 15,17 by a welding seal line 19. The small upper compartment 15 comprises a concentrated glucose solution having a concentration of about 50%. The large lower compartment 17 comprises all other electrolytes necessary for the solution to be formed, such as NaCl, MgCl₂, CaCl₂, lactate etc, dissolved in water in a manner known per se. If the bag should be used as a nutritional solution, the large compartment may comprise only NaCl or any suitable composition as used today but excluding glucose. The entire bag filled with solution is sterilized, preferably by autoclaving at 120°C during 20 minutes.

It is mentioned that the glucose can be exchanged with a glucose-like compound, such as glucose polymers, as an osmotic agent.

In order to obtain access to compartments 15 and 17 during the manufacturing process, the borders 3,5 are perforated by introduction tubes 21. Such tubes 21 are inserted in borders 3,5 during the welding procedure. Initially, tubes 21 are long enough and/or provided with connection means to be automatically connected to a filling equipment of the manufacturing plant. After filling compartments 15,17, the tubes are cut and sealed by heat as shown by end portions 23 in Fig. 1.

The lower border 5 is also provided with an outlet tube 25, which connects compartment 17 with the consumer, for example a catheter ending in the abdominal cavity of a patient for peritoneal dialysis. Often tube 25 terminates in a luer connector (not shown in Fig. 1).

Furthermore, border 5 is provided with a medicament tube 27 including a removable cap 29. When cap 29 is removed, tube 27 forms an entrance site for introducing any type of beneficial agent or medicament into compartment 17 as desired, such as antibiotica.

Welding line 19 is provided with a connection tube 31 initially closed by a breakable portion 33. When portion 33 is broken, communication is established between compartment 15 and compartment 17. While compartment 15 comprises glucose of high concentration having a high density, the glucose will flow down through tube 31 into the lower compartment 17 and mix with the contents thereof. After a few minutes, compartment 15 is completely empty. Any residual fluid can be forced out from compartment 15 manually.

As appears from Fig. 1, tube 31 is placed in line with introduction tube 21. The reason for this is that it is more easy to insert tubes 21 and 31 if they are in line during the manufacturing step. Both tubes are attached to a mandrel interposed in the bag blank before welding, whereupon the welding step is performed. After welding, the mandrel is withdrawn. Preferably, the welding takes place by high frequency welding but of course any other type of heat welding or sealing (glueing) would be suitable depending on the material.

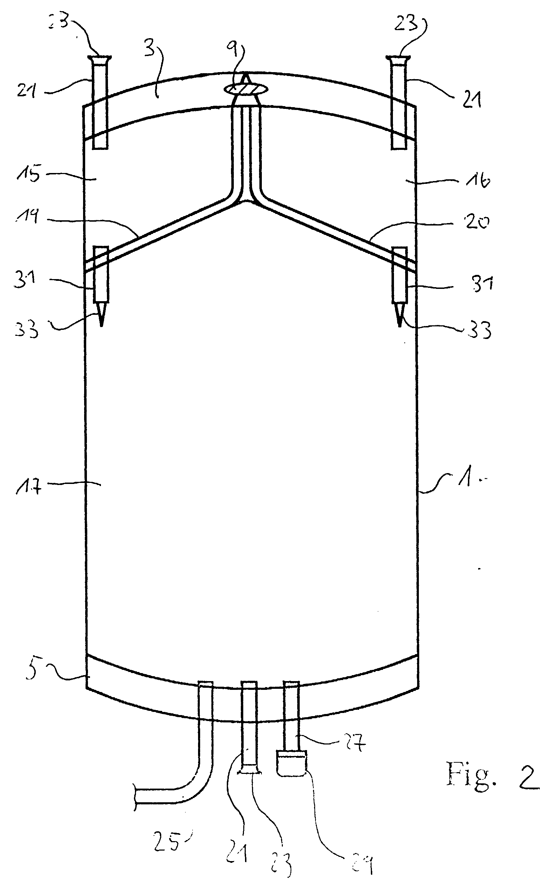

A dialysis solution for peritoneal dialysis is usually provided in three different concentrations, viz. 1,5%, 2,5%, 4% of glucose concentration in the final solution. By using a three compartment bag, previously known per se, all three concentration levels can be obtained in a single bag. In this first embodiment, shown in Fig. 2, of the present invention, the upper compartment is divided into two compartments of equal size by two sloping welding lines 19,20. Thus, there is formed a first upper compartment 15 and a second upper compartment 16, each being accessed via introduction tubes 21. The first upper compartment 15 comprises glucose solution having a concentration of about 30% and the second upper compartment 16 comprises a glucose solution having a concentration of about 50%.

When breaking the breakable portion 33 of connection tube 31, the contents of the first upper compartment 15 is mixed with the contents of the lower compartment 17 to form a peritoneal dialysis solution having a concentration of 1,5% of glucose. If the breakable portion of connection tube 31 is broken, the contents of compartment 16 is mixed with the contents of compartment 17 thereby forming a dialysis solution having a concentration of about 2,5% of glucose. If both breakable portions 33 of connection tubes 31 are broken, the contents of both compartments 15 and 16 are mixed with the contents of compartment 17 thereby to form a dialysis solution having a concentration of about 4% of glucose.

In this way it is only necessary to store one type of bag for all three concentrations. While one and the same patient may use solutions of different concentrations during different times of the day or different days, the operation for the patient will also be improved. Other combinations of concentration levels can easily be achieved. Further different concentration can be obtained by including further compartments, such as four or five compartments.

It is noted that some glucose is wasted when the bag is used for the two lower concentrations, since one of the two glucose compartments is left unused. However, glucose is an inexpensive material. Moreover, glucose can easily be discarded, since it does not harm the environment to any large extent. The advantages of providing one bag for all three concentrations in economical terms and handling terms far outweigh this drawback.

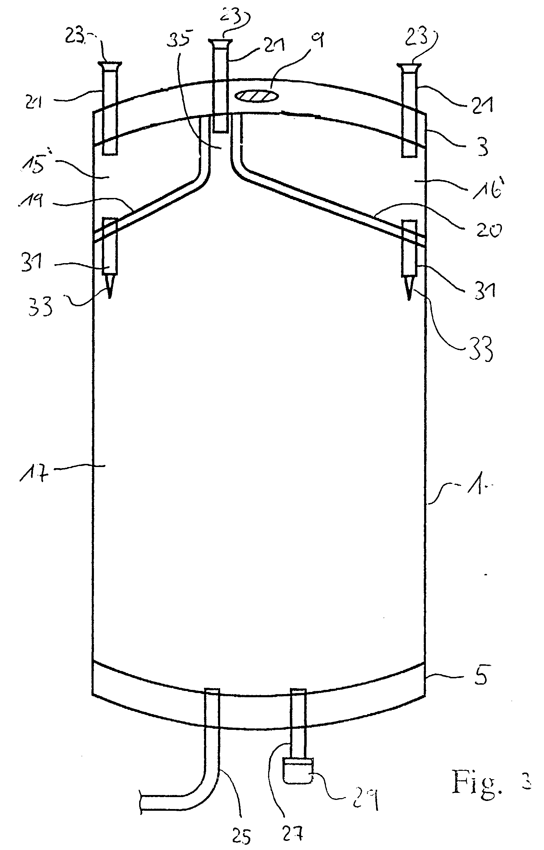

Fig. 3 shows another embodiment of the invention which very much resembles the embodiment shown in Fig. 2. The first upper compartment 15' and the second upper compartment 16' have different sizes but accomodated glucose solution of the same concentration, for example 50%. Thus, the same possibilities appear as from the first embodiment shown in Fig. 2.

One example of a PD solution according to the embodiment of Fig. 3 is given below. The first upper compartment 15' comprises 60 ml glucose at 50% and the second upper compartment 16' comprises 100 ml glucose of 50%, both at a pH of 3-3,5.

The lower compartment 17 comprises 1900 ml of electrolytes having a pH of about 6,5. The electrolytes are: 264 mMoles Na⁺, 190 mMoles Cl⁻, 2,7 mMoles Ca⁺⁺, 0,5 mMoles Mg⁺⁺ and 80 mMoles lactate. After mixing, the lower compartment 17 includes a solution having 1,53%, 2,5% or 3,88% glucose concentration and the following electrolytes concentrations: Na⁺ 128-135 mM, Cl⁻ 92-97 mM, Ca⁺⁺ 1,31-1,38 mM, Mg⁺⁺ 0,24-0,26 mM and lactate 38-41 mM, depending on which compartments have been mixed.

Moreover, the embodiment according to Fig. 3 is provided with a channel 35 positioned between welding line 19 forming the first compartment 15' and welding line 20 forming the second compartment 16' as clearly shown in Fig. 3. The upper border 3 of the bag is provided with two introduction tubes 21 for introduction of the glucose solution. Moreover, border 3 is provided with a third introduction tube 21 ending in channel 35 for providing the electrolyte solution into compartment 17. The second lower border 5 is only provided with a tube 27 for introduction of a medicament and a tube 25 for connection to the patient but lacks an introduction tube shown in the previous embodiment.

By having all three introduction tubes 21 in the upper border 3, several manufacturing advantages are obtained since all filling equipment now can be placed on one side of the manufacturing line.

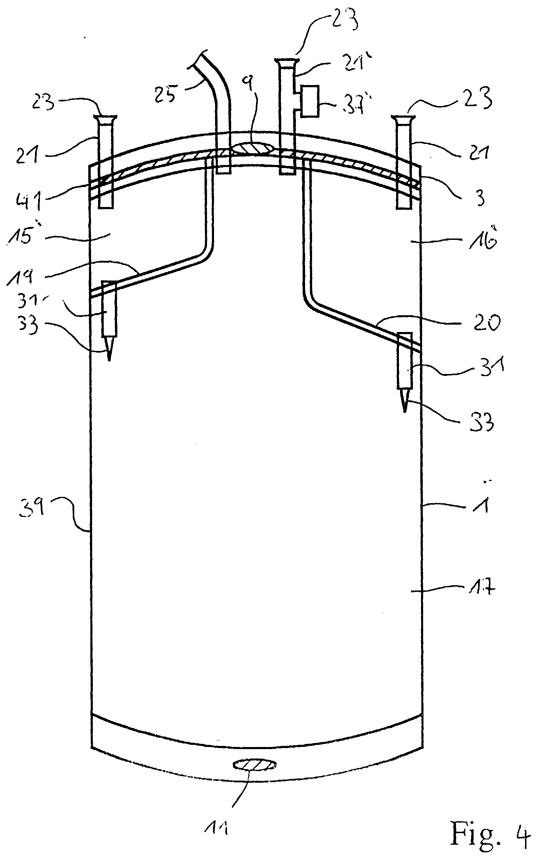

It is realized that the different features shown on the different embodiments can be combined in different manners to obtain desired benefits and requirements. Further embodiments are possible, such as including all tubes in a single border. Such an embodiment is shown in Fig. 4. The upper border 3 comprises two introduction tubes 21 ending in first upper compartment 15' and second upper compartment 16' for introduction of a glucose solution of a high concentration therein. Compartments 15' and 16' are delimited by welding lines 19,20 leaving the central portion of border 3 open towards the large bottom compartment 17. In the central portion of border 3 is placed an outlet tube 25 and a combined introduction tube 21' and medicament introduction port 37'.

Since all connections are included in border portion 3, it can be made as a single separate injection moulded part, to which the bag blank 39 is welded as shown by welding lines 41.

When using the bag according to Fig. 4, it is supported in the position shown in Fig. 4 via aperture 9 and the respective breakable portions 33 are broken to mix the contents of compartment 15' and/or 16' with the contents of compartment 17. When the mixing procedure is ended, the bag is reversed and supported from the other direction from the aperture 11, whereupon outlet tube 25 will be positioned in the lowermost part of the bag. In this way, the mixing of the different solutions will be further improved.

Such a moulded part can have sealing surfaces to the manufacturing and filling equipment which makes it more convenient to the filling procedure. The moulded part can include grip portions for easy handling during storage and use etc.

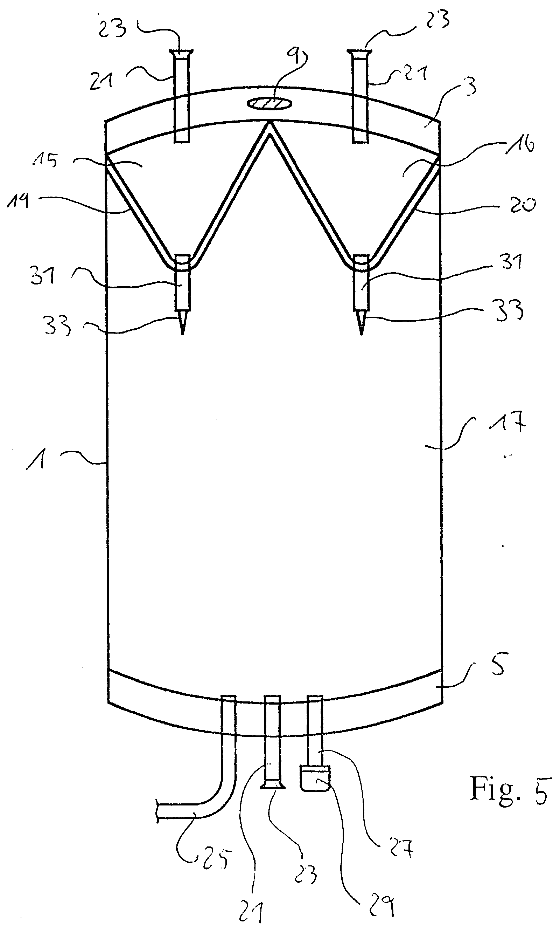

Another variant of the present invention is shown in Fig. 5. The bag according to Fig. 5 comprises three compartments formed by two V-shaped welding lines 19,20. In other respects this embodiment is similar to the embodiment shown in Fig. 2.

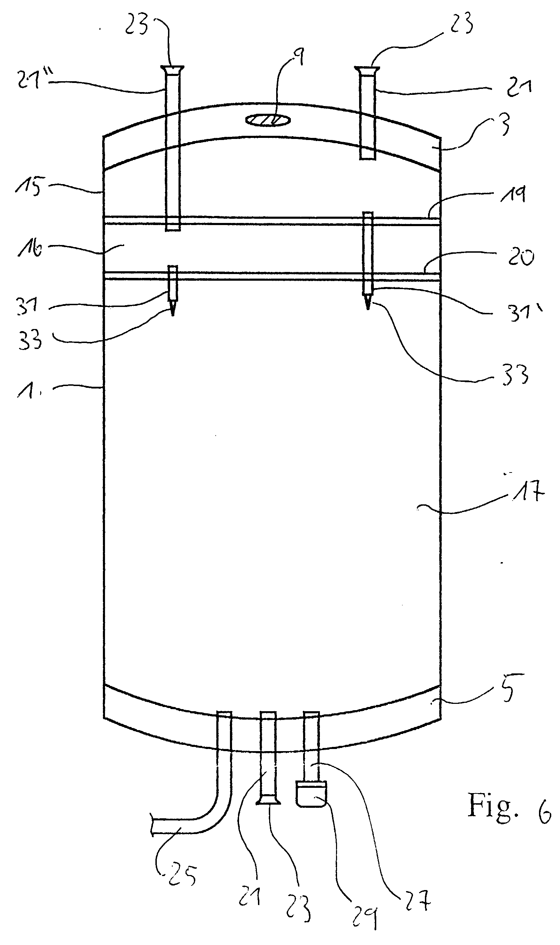

Fig. 6 shows a variant of the embodiment according to Fig. 2 and includes three compartments 15,16 and 17 formed by welding lines 19 and 20. The upper compartment 15 is filled with glucose solution via introduction tube 21 and is connected to the large bottom compartment 17 via a connection tube 31' extending from the upper compartment 15 beyond the intermediate compartment 16 to the lower compartment 17. The second compartment 16 is filled with glucose solution via introduction tube 21'' extending from the upper border edge 3 through compartment 15 to compartment 16. Compartment 16 is connected to compartment 17 via connection tube 31. In all other respects this embodiment is similar to the embodiment according to Fig. 2.

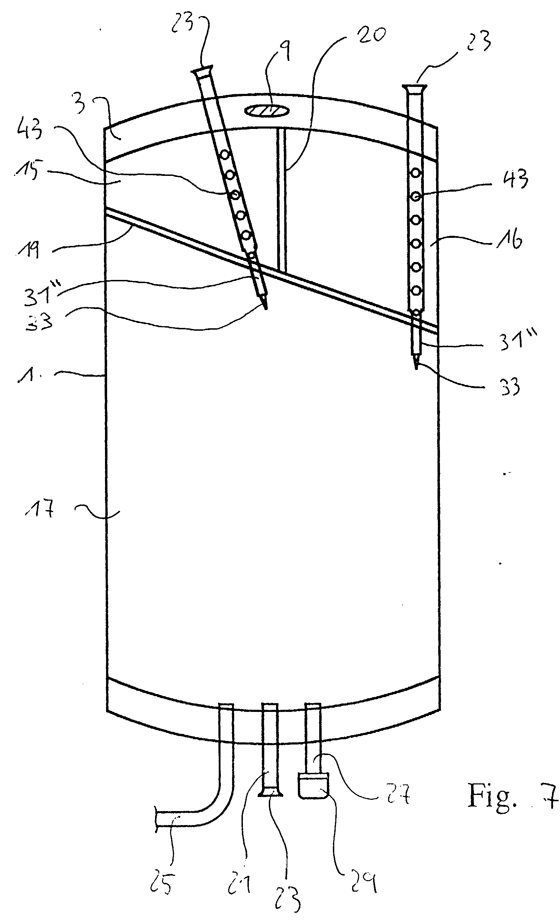

Finally, Fig.7 shows another embodiment of the invention having three compartments 15,16 and 17. A welding line 19 limits the large compartment 17 comprising electrolyte solution from compartments 15 and 16 comprising glucose solution. Welding line 19 is inclined and comprises two connection tubes 31''. The operation is similar to the embodiment according to Fig. 2.

Each connection tube 31'' is provided with several holes 43 connecting tube 31'' to the compartment 15,16 for glucose, and a break pin 31 connecting tube 31'' with the large compartment 17. In this way the connecting tube operates both as an introcution tube similar to tubes 21 of Fig. 2 and as a connection tube similar to tubes 31 of Fig. 2. Thus, a smaller number of tubes are required.

Several different embodiments of the invention have been described above with reference to Fig. 2-7. The different detailed constructions of each embodiment can be combined in further different ways.

It is realized that the bag will have different sizes depending on the intended field of use. The concentration and other features of the glucose-part can be different depending on the actual use, such as 20% - 50% or even more.

Herein above, several embodiments of the invention have been described in the purpose of exemplifying the invention. The invention is only limited by the appended patent claims.

## Claims

1. Container for enclosing a sterile medical solution containing glucose, for example a solution intended for peritoneal dialysis, comprising:
a first compartment (17) having a size sufficiently large for accomomodating the sterile medical solution; and
at least two further compartments (15,16;15',16') separated from each other and from the first compartment by separation lines (19,20),
**characterized** in that
said further compartments (15,16,15',16') all comprise glucose at a high concentration, for example above about 20%; and
said first compartment comprising the remaining portion of the sterile medical solution comprising an electrolyte solution, for example NaCl, CaCl₂, etc.; and
connection means (31,31',31'',33) for selectively connecting said further compartments with said first compartment for mixing the contents of said further compartments with the contents of said first compartment for producing said sterile medical solution in at least three optionally selectable different concentrations of glucose.

2. Container according to claim 1, **characterized** in that said further compartments comprise a second compartment (15) and a third compartment (16) having the same volume and enclosing glucose in different concentrations, for example 30% and 50%, respectively.

3. Container according to claim 2, **characterized** in that said third compartment (16') has a larger volume than said second compartment (15') and in that the second and third compartments comprise glucose at the same high concentration, for example 50%.

4. Container according to claim 3, **characterized** in that said second compartment (15') comprises 60 ml glucose at 50%, said third compartment (16') comprises 100 ml glucose at 50%, and said first compartment (17) comprises 1900 ml of electrolytes comprising 262 mMoles Na⁺, 190mMoles Cl⁻, 2,7 mMoles Ca⁺⁺, 0,5 mMoles Mg⁺⁺ and 80 mMoles lactate.

5. Container according to anyone of the preceding claims, **characterized** in that the separation lines (19,20) between the first compartment (17) and each of said further compartments (15,16,15',16') are inclined when the container is placed in a first mixing position wherein said further compartments are positioned above said first compartment.

6. Container according to claim 5, **characterized** by a first border edge (3) comprising introduction tubes (21,21',21'') for introducing solution in said compartments.

7. Container according to claim 6, **characterized** in that said first border edge (3) is made of a single separate part.

8. Container according to claim 7, **characterized** in that said first border edge (3) comprises three introduction tubes (21), one for each compartment (15,16,15',16',17).

9. Container according to anyone of the preceding claims, **characterized** in that said connection means is a connection tube (31,31',31'') including breakable means (33) and establishing fluid communication there through at activation of said breakable means.

10. Method for mixing sterile medical solution into at least three optionally selectable different concentrations of glucose, for example a solution intended for peritoneal dialysis, including the steps of:
providing a container including a first compartment (17) having a size sufficiently large for accommodating the sterile medical solution; and at least two further compartments (15,16) separated from each other and from the first compartment by separation lines (19,20), said further compartments (15,16) all comprise glucose at a high concentration, for example above about 20%; and said first compartment comprising the remaining portion of the sterile medical solution comprising an electrolyte solution, for example NaCl, CaCl₂, etc.;
providing fluid communications between one of said further compartments and said first compartment for mixing the contents thereof for providing a peritoneal dialysis solution having a first low concentration, for example about 1,5%, or a second intermedicate concentration, for example about 2,5%, respectively of glucose; and
optionally providing fluid communication between another of said further compartments and said first compartment for providing a peritoneal dialysis solution having a third, higher concentration of glucose, for example about 4%.

11. Use of a container including a first compartment (17) having a size sufficiently large for accommodating a sterile medical solution; and at least two further compartments (15,16) separated from each other and from the first compartment by separation lines (19,20), said further compartments (14,15) all comprise glucose at a high concentration, for example above about 20%, and said first compartment comprising the remaining portion of the sterile medical solution comprising an electrolyte solution, for example NaCl, CaCl₂, etc., for providing said sterile medical solution in at least three optionally selectable different concentrations of glucose, for example a solution intended for peritoneal dialysis, by mixing the contents of the first compartment with the contents of one or several of said further compartments.

## Patentansprüche

1. Behälter zum Einschließen einer Glucose enthaltenden sterilen medizinischen Lösung, wie beispielsweise einer Lösung für Peritonealdialyse, mit
einem ersten Abteil (17) mit einer ausreichenden Größe zur Aufnahme der sterilen medizinischen Lösung und
wenigstens zwei weiteren Abteilen (15, 16; 15', 16'), die voneinander und von dem ersten Abteil durch Trennlinien (19, 20) getrennt sind,
**dadurch gekennzeichnet**, daß
diese weiteren Abteile (15, 16; 15', 16') alle Glucose mit einer hohen Konzentration, wie beispielsweise oberhalb etwa 20 %, umfassen und
das erste Abteil den restlichen Anteil der sterilen medizinischen Lösung mit einer Elektrolytlösung, wie beispielsweise NaCl, CaCl₂ usw. umfaßt, und
eine Verbindungseinrichtung (31, 31', 31", 33) zur selektiven Verbindung der weiteren Abteile mit dem ersten Abteil vorgesehen ist, um den Inhalt der weiteren Abteile mit dem Inhalt des ersten Abteils zu vermischen und so die sterile medizinische Lösung in wenigstens drei gegebenenfalls auswählbaren unterschiedlichen Glucosekonzentrationen zu erzeugen.

2. Behälter nach Anspruch 1, **dadurch gekennzeichnet**, daß die weiteren Abteile ein zweites Abteil (15) und ein drittes Abteil (16) mit dem gleichen Volumen und einem Gehalt an Glucose in unterschiedlichen Konzentrationen, wie beispielsweise 30 bzw. 50 %, umfassen.

3. Behälter nach Anspruch 2, **dadurch gekennzeichnet**, daß das dritte Abteil (16') ein größeres Volumen als das zweite Abteil (15') hat und das zweite und dritte Abteil Glucose mit der gleichhohen Konzentration, wie beispielsweise 50 %, umfassen.

4. Behälter nach Anspruch 3, **dadurch gekennzeichnet**, daß das zweite Abteil (15') 60 ml 50 %ige Glucose, das dritte Abteil (16') 100 ml 50 %ige Glucose und das erste Abteil (17) 1900 ml Elektrolyte umfassen, die 262 mMol Na⁺, 190 mMol Cl⁻, 2,7 mMol Ca⁺⁺, 0,5 mMol Mg⁺⁺ und 80 mMol Lactat enthalten.

5. Behälter nach einem der vorausgehenden Ansprüche, **dadurch gekennzeichnet**, daß die Tennlinien (19, 20) zwischen dem ersten Abteil (17) und jedem der weiteren Abteile (15, 16; 15', 16') geneigt sind, wenn der Behälter in eine erste Mischposition gebracht wird, in der die weiteren Abteile oberhalb des ersten Abteils angeordnet sind.

6. Behälter nach Anspruch 5, **gekennzeichnet durch** eine erste Randkante (3), die Einführschläuche (21, 21', 21") zur Einführung von Lösung in die Abteile umfaßt.

7. Behälter nach Anspruch 6, **dadurch gekennzeichnet**, daß die erste Randkante (3) aus einem einzelnen getrennten Teil besteht.

8. Behälter nach Anspruch 7, **dadurch gekennzeichnet**, daß die erste Randkante (3) drei Einführschläuche (21) umfaßt, einen für jedes Abteil (15, 15; 15', 16'; 17).

9. Behälter nach einem der vorausgehenden Ansprüche, **dadurch gekennzeichnet**, daß die Verbindungseinrichtung ein Verbindungsschlauch (31, 31', 31") ist, der wegbrechbare Einrichtungen (33) einschließt und eine Fluidverbindung dort hindurch bei Aktivierung der wegbrechbaren Einrichtung herstellt.

10. Verfahren zum Mischen einer sterilen medizinischen Lösung in wenigstens drei gegebenenfalls auswählbaren unterschiedlichen Glucosekonzentrationen, wie beispielsweise einer Lösung für Peritonealdialyse, mit den Stufen, in denen man
einen Behälter vorsieht, der ein erstes Abteil (17) mit einer ausreichenden Größe zur Aufnahme der sterilen medizinischen Lösung und wenigstens zwei weitere Abteile (15, 16), die voneinander und von dem ersten Abteil durch Trennlinien (19, 20) getrennt sind, einschließt, wobei diese weiteren Abteile (15, 16) alle Glucose mit einer hohen Konzentration, wie beispielsweise oberhalb etwa 20 %, umfassen und das erste Abteil den restlichen Anteil der sterilen medizinischen Lösung enthält, welche eine Elektrolytlösung, wie beispielsweise NaCl, CaCl₂ usw., umfaßt,
Fluidverbindungen zwischen einem dieser weiteren Abteile und dem ersten Abteil herstellt, um deren Inhalte zu vermischen und so eine Peritonealdialyselösung zu bekommen, die eine erste niedrige Konzentration, wie beispielsweise etwa 1,5 %, bzw. eine zweite mittlere Konzentration, wie beispielsweise etwa 2,5 %, Glucose hat, und gegebenenfalls eine Fluidverbindung zwischen einem anderen dieser weiteren Abteile und dem ersten Abteil herstellt, um eine Peritonealdialyselösung mit einer dritten höheren Glucosekonzentration, wie beispielsweise etwa 4 %, zu bekommen.

11. Verwendung eines Behälters mit einem ersten Abteil (17) mit einer ausreichenden Größe, um eine sterile medizinische Lösung aufzunehmen, und mit wenigstens zwei weiteren Abteilen (15, 16), die voneinander und von dem ersten Abteil durch Trennlinien (19, 20) getrennt sind, wobei diese weiteren Abteile (14, 15) alle Glucose mit einer hohen Konzentration, wie beispielsweise oberhalb etwa 20 %, umfassen und das erste Abteil den restlichen Anteil der sterilen medizinischen Lösung mit einer Elektrolytlösung, wie beispielsweise NaCl, CaCl₂ usw., umfaßt, um die sterile medizinische Lösung in wenigstens drei gegebenenfalls auswählbaren unterschiedlichen Glucose-konzentrationen, wie beispielsweise einer Lösung für Peritonealdialyse, zu erhalten, indem man den Inhalt des ersten Abteils mit den Inhalten eines oder mehrerer der anderen Abteile vermischt.

## Revendications

1. Récipient pour renfermer une solution médicale stérile contenant du glucose, par exemple une solution destinée à une dialyse péritonéales comprenant :
un premier compartiment (17) ayant une dimension suffisamment grande pour accepter la solution médicale stérile ; et
au moins deux compartiments supplémentaires (15, 16 ; 15', 16') séparés l'un de l'autre et du premier compartiment par des cloisons de séparation (19, 20),
caractérisé en ce que
lesdits compartiments supplémentaires (15, 16 ; 15', 16') comprennent tous du glucose à une concentration élevée, par exemple supérieure à environ 20 % ; et
ledit premier compartiment comportant la partie restante de la solution médicale stérile comprenant une solution d'électrolyte, par exemple NaCl, CaCl₂, etc. ; et
des moyens de liaison (31, 31', 31", 33) pour sélectivement relier lesdits compartiments supplémentaires avec ledit premier compartiment afin de mélanger les contenus desdits compartiments supplémentaires avec les contenus du premier compartiment pour produire ladite solution médicale stérile dans au moins trois concentrations de glucose différentes sélectionnables optionnellement.

2. Récipient selon la revendication 1, caractérisé en ce que lesdits compartiments supplémentaires comprennent un deuxième compartiment (15) et un troisième compartiment (16) ayant le même volume et renfermant du glucose à différentes concentrations, par exemple 30 % et 50 % respectivement.

3. Récipient selon la revendication 2, caractérisé en ce que ledit troisième compartiment (16') présente un volume plus grand que ledit deuxième compartiment (15') et en ce que les deuxième et troisième compartiments comprennent du glucose à la même concentration élevée, par exemple 50 %.

4. Récipient selon la revendication 3, caractérisé en ce que le deuxième compartiment (15') comprend 60 ml de glucose à 50 %, le troisième compartiment (16') comprenant 100 ml de glucose à 50 %, et ledit premier compartiment (17) comprenant 1900 ml d'électrolyte comportant 262 mMoles Na⁺, 190 mMoles Cl⁻, 2,7 mMoles Ca⁺⁺, 0,5 mMoles Mg⁺⁺ et 80 mMoles de lactate.

5. Récipient selon l'une quelconque des revendications précédentes, caractérisé en ce que les cloisons de séparation (19, 20) entre le premier compartiment (17) et chacun desdits compartiments supplémentaires (15, 16, 15', 16') sont inclinées lorsque le récipient est disposé dans une première position de mélange dans laquelle lesdits compartiments supplémentaires sont positionnés au-dessus dudit premier compartiment.

6. Récipient selon la revendication 5, caractérisé en ce qu'il comprend un premier bord d'extrémité (3) comportant des tubes d'introduction (21, 21', 22") pour introduire une solution dans lesdits compartiments.

7. Récipient selon la revendication 6, caractérisé en ce que le premier bord d'extrémité (3) est réalisé dans une même partie séparée.

8. Récipient selon la revendication 7, caractérisé en ce que le premier bord d'extrémité (3) comprend trois tubes d'introduction (21), un pour chaque compartiment (15, 16, 15', 16', 17).

9. Récipient selon l'une quelconque des revendications précédentes, caractérisé en ce que les moyens de liaison sont constitués par un tube de liaison (31, 31', 31") incluant des moyens cassables (33) et établissant une communication fluidique lors de l'activation des moyens cassables.

10. Procédé pour mélanger une solution médicale stérile dans au moins trois concentrations différentes de glucose sélectionnables optionnellement, par exemple une solution destinée à une dialyse péritonéale, comportant les étapes de :
fournir un récipient comprenant un premier compartiment (17) présentant une dimension suffisamment grande pour accepter la solution médicale stérile ; et au moins deux compartiments supplémentaires (15, 16) séparés l'un de l'autre et du premier compartiment par des lignes de séparation (19, 20), lesdits compartiments supplémentaires (15, 16) comprenant tous du glucose à une concentration élevée par exemple supérieure à environ 20 % ; ledit premier compartiment comportant la partie restante de la solution médicale stérile comprenant une solution d'électrolyte, par exemple NaCl, CaCl₂, etc. ;
ménager des communications fluidiques entre un desdits compartiments supplémentaires et ledit premier compartiment pour mélanger leurs contenus de manière à fournir une solution de dialyse péritonéale présentant une première faible concentration, par exemple environ 1,5 %, ou une deuxième concentration intermédiaire, par exemple environ 2,5 %, respectivement de glucose ; et
ménager optionnellement une communication fluidique entre un autre desdits compartiments supplémentaires et ledit premier compartiment afin de disposer d'une solution de dialyse péritonéale présentant une troisième concentration de glucose plus élevée, par exemple environ 4 %.

11. Utilisation d'un récipient comportant un premier compartiment (17) ayant une dimension suffisamment grande pour accepter une solution médicale stérile ; et au moins deux compartiments supplémentaires (15, 16) séparés l'un de l'autre et du premier compartiment par des cloisons de séparation (19, 20), lesdits compartiments supplémentaires (15, 16) comprenant tous du glucose à une concentration élevée, par exemple supérieure à environ 20 %, le premier compartiment comportant la partie restante de la solution médicale stérile comprenant une solution d'électrolyte, par exemple NaCl, CaCl₂, etc. ; afin de disposer de ladite solution médicale stérile dans au moins trois concentrations différentes de glucose sélectionnables optionnellement, par exemple une solution destinée à une dialyse péritonéale, en mélangeant les contenus du premier compartiment avec les contenus d'un ou plusieurs desdits compartiments supplémentaires.
